(19) Europäisches Patentamt
European Patent Office
Office européen des brevets

(11) Publication number: **0 097 506 A2**

# (12) EUROPEAN PATENT APPLICATION

(21) Application number: 83303520.7

(22) Date of filing: 17.06.83

(51) Int. Cl.³: C 12 Q 1/34
G 01 N 33/04

(30) Priority: 21.06.82 AU 4520/82

(43) Date of publication of application: 04.01.84 Bulletin 84/1

(84) Designated Contracting States: DE FR GB NL SE

(71) Applicant: Ahern, Michael John
C/- Department of Primary Industries Cnr. George and Ann Streets
Brisbane Queensland 4001(AU)

(72) Inventor: Kitchen, Barry James Dept. of Primary Industries
Dairy Research Laboratory 9 Hercules Street
Hamilton Queensland, 4007(AU)

(74) Representative: Brown, John David et al,
FORRESTER & BOEHMERT Widenmayerstrasse 4/I
D-8000 München 22(DE)

## (54) Method for diagnosis of mastitis using enzyme N-acetyl-beta-D-glucosaminidase and reaction systems for use in method.

(57) A method for diagnosis of mastitis in cattle by a colorimetric determination of the enzyme N-ACETYL-β-D-GLUCOSAMINIDASE (NAGase) in milk samples. The method comprises -

(1) adding a milk sample to a preheated aqueous substrate/buffer solution comprising a substrate having the formula

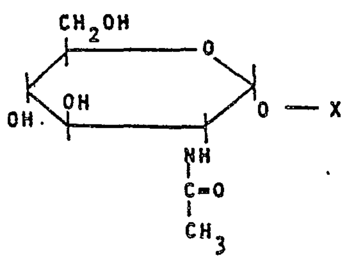

and an acid/anion buffer,

(2) incubating the mixture for a suitable period then stopping the reaction,

(3) conducting a colorimetric comparison between sample and standard.

The invention also includes a test kit for carrying out the method.

EP 0 097 506 A2

THIS INVENTION relates to a method for diagnosis of mastitis using the enzyme N-acetyl-B-D-glucosaminidase (hereinafter called NAGase) and reaction systems including reaction kits for use in the method.

It is reported in successive Journals of Dairy Research in 1976 (ie. Volume 43 pages 251-8, volume 43, pages 491-4, and Volume 45 pages 15-20) that diagnosis of mastitis can now be carried using the enzyme NAGase. This matter is also reported in the Journal of Dairy Research (63, 978-83).

However, while the assay procedures described in the abovementioned publications have been useful in demonstrating the effectiveness of using the enzyme in diagnosis of mastitis, there has now been developed a suitable reaction system and associated method using this enzyme which may be used by semi-skilled people such as farm workers and stock agents or skilled people such as veteriarians. Use of the reaction system of this invention makes it now possible to detect mastitis "in situ" without it being necessary to forward milk samples for testing to laboratories for analysis.

The reaction system of the invention includes the following components;

(A) a substrate for reaction with enzyme NAGase selected from a compound of the formula

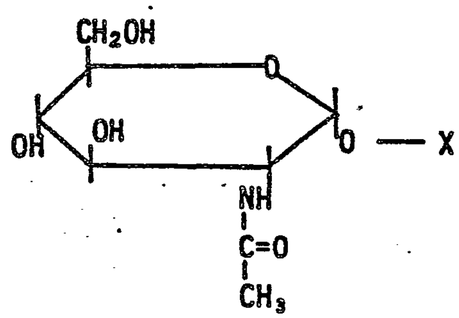

where X is selected from p-nitrophenol, α-naphthol, p-nitrocatechol. 3-3 bis (p-hydroxyphenyl) phthalide, 2-methoxy-4-(2'-nitrovinyl)-phenyl, and 4 methyl umbelliferone, and

(B) a buffer for substrate (i) to maintain the pH of the substrate within the range 3.5 to 6.0, said buffer being in the form of an acid/anion mixture preferably selected from citric acid/citrate, acetic acid/acetate, formic acid/formate and lactic acid/lactate.

Preferably component A is present in the reaction mixture in an amount of 0.5 to 2.5%, the buffer anion is present in an amount of from 50 to 75.0% and the buffer acid is present in an amount of 20 to 40%. These amounts are calculated on the basis that the reaction mixture is present in 100 g of $H_2O$ and that for each volume of reaction mixture there also is used an equal volume of milk in the testing procedure. Preferred ranges for component A are 1.0 to 1.5% (more preferably 1.3%). A preferred range for the buffer anion is from 65-70% (more preferably 68.0%) and for the buffer acid is from 25-35% (more preferably 30.0%).

The reaction system stops the reaction between substrate and enzyme by raising the pH to a value of between 8 and 11. Suitable stopping agents include EDTA (eg. the sodium salt thereof) glycine, sodium hydroxide, sodium borate and sodium carbonate. The stopping agent may be present in any suitable amount.

The reaction system may also include appropriate standards and these may comprise a compound selected from p-nitrophenol, α-naphthol, p-nitrocatechol, 3-3 bis (p-hydroxyphenyl)-phthalide, 2-methoxy-4-(2'-nitrovinyl)-phenyl or 4 methyl umbelliferone in suitable proportions. Suitably the standard solutions may comprise a plurality of a diluted basic or starting standards so as to form a range of colour standards to which a test solution may be compared.

The abovementioned reaction system suitably is in the form of a kit containing predetermined (eg. usually on the basis of weight) amounts of substrate-buffer, standards and stopping agent. The system may be marketed with separate vials containing each of the abovedescribed components.

The manually operated method of the invention may include the following steps:

(i) addition of water to substrate-buffer vial or support medium followed by heating,

(ii) addition of milk sample to reaction vessel, containing substrate-buffer vial or support medium.

(iii) incubating for suitable period, and

(iv) addition of stopping agent followed by reading colour in a colour comparator vessel or against the prepared set of standards.

In relation to the substrates described above, p-nitrophenol produces a yellow colour, α-naphthol produces a red colour, p-nitrocatechol produces a yellow-orange colour, 3-3-bis (p-pydroxyphenol)-phthalide produces a pink-red colour and 4-methyl umbelliferone is detectable in UV light producing a blue fluorescence. 2-methoxy-4-(2'nitrovinyl)-phenyl produces a red colour.

It will also be appreciated that the above described components of the reaction system of the invention may be varied by including separate vials of substrate and buffer so that a kit could be marketed including a substrate vial, buffer vial and optionally the other components such as the stopping agent vial and the standards. Suitable examples of reaction systems and methods utilizing same are described below.

EXAMPLE 1

A reaction system of components (a), (b) and (c) is prepared as set out below.

(a) Substrate Vial - 10 per kit.

Each vial upon addition of 12 mls. of $H_2O$ would produce a substrate concentration of 4mM in 0.4M citrate pH 4.5. The required amounts for this are as follows:

16.4 mg. of p-NP-NAG
0.80 g. tri-sodium citrate ($2H_2O$)
0.40 g. citric acid ($1.H_2O$)
} 12 mls. of $H_2O$

Each vial would be sufficient for 48 tests and the complete kit would do 480 tests.

(b) Standard Vial - 1 per kit

The vial would contain 10.5 mg. of p-nitrophenol which would be reconstituted with 100 mls. of $H_2O$. This would be the stock

standard solution. This would then be diluted 10 fold with 0.1M solium carbonate buffer pH 10. This diluted standard (called solution A) is further diluted as follows to produce a range of colour standards with which test solution can be compared (a scoring system of 1 to 10 can be used).

PREPARATION OF STANDARDS

| Standard No.** | Mls. of * Milk | Mls. of Soln. (A) | Mls. of 0.1M pH 10 Carbonate | Comparator Reading |
|---|---|---|---|---|
| 1 | 0.25 | 0 | 5.75 | 0 |
| 2 | 0.25 | 0.3 | 5.45 | 6 |
| 3 | 0.25 | 0.5 | 5.25 | 10 |
| 4 | 0.25 | 0.8 | 4.95 | 14 |
| 5 | 0.25 | 1.0 | 4.75 | 18 |
| 6 | 0.25 | 1.5 | 4.25 | 25 |
| 7 | 0.25 | 2.5 | 3.25 | 42 |
| 8 | 0.25 | 3.0 | 2.75 | 42 |
| 9 | 0.25 | 4.0 | 1.75 | 42 |
| 10 | 0.25 | 5.0 | 0.75 | 42 |

* Any reasonably fresh milk (cream line) can be used.

** These numbers can be used to give a score from 1 to 10.

This set of standards can be used either independently or in conjunction with measurements taken on test solutions by a "LOVIBOND" comparator. (Alkaline phosphatase disc APTW 241-300)

(c) Stopping Reagent Vial - 10 per kit.
Each vial contains 2.5 g of $Na_2CO_3$ which is dissolved in 250 mls. of $H_2O$. Sufficient for 48 tests. Alternatively 5g of $Na_2$ EDTA could be used dissolved in 250 ml. of $H_2O$.

OTHER EQUIPMENT NEEDED

(i) Test tubes - size (15 mm O.D.)
(ii) Pipettes
(iii) Water bath 45°C
(iv) "LOVIBOND" Comparator and suitable disc (Alkaline phosphatase APTW) - optional.

METHOD

(I) Add 12 mls, of $H_2O$ to substrate vial. (Warm if necessary). Place in 45°C water bath.

(II) Dissolve stopping reagent in 250 mls. of $H_2O$.

(III) Pipette 0.25 mls. of milk sample into test tube and place in water bath for 2-3 mins.

(IV) Add 0.25 mls. of warm (25°C) substrate solution and mix well. (slight shaking).

(V) Incubate for 15 mins. * at 45°C.

(VI) Then after 15 mins. * add 5.5 mls. of stopping solution.

(VII) Read colour in a comparator or against the prepared set of standards.

* Use 15 mins. for quarter fore-milk samples and 30 mins. for farm bulk milks or road tanker samples and composite samples.

INTERPRETATION OF RESULTS

Quarter Samples: (15 min. assay)

The best approach for quarter samples is to observe if there are any differences between a set of 4 quarters. One or two higher readings indicates a problem animal and the positive quarters can easily be located.

| Comparator Reading | Interpretation |
|---|---|
| 0,6 | Negative |
| 10 | Suspect |
| 14,18,25,42 | Positive |

Composite Samples: (30 min. assay)

| | |
|---|---|
| 0, 6, 10 | Negative |
| 14 | Suspect |
| 18,25,42 | Positive |

Bulk Samples: (30 min. assay)

| | |
|---|---|
| 0,6,10 | Farms with minimum mastitis problems |
| 14 | Suspect |
| 18,25,42 | Farms with definite problems |

REAGENT STABILITY

(a) Substrate:

Reconstituted reagent is stable for up to 2 weeks at 5°C. If possible protect from direct light. Reagent stored at 25°C is stable for 2-3 days (if protected from light). Dried reagent in vials are stable for at least 6 months at 5°C.

(b) Standard:

Indefinitely stable in solid form at 5°C (if protected from direct light), while stable for up to 3 months at 5°C when reconstituted in $H_2O$.

(c) Stopping material:

Stable indefinitely in solid form and for at least 12 months at room temperature in reconstituted form.

READING RESULTS

Suitable lighting is required in order to efficiently read the intensity of the yellow colour produced.

EXAMPLE 2

In an alternative procedure to that described above in Example 1 a reaction kit can be devised that is in accordance with the invention to provide a basis for rapid testing on the farm so that "problem" cows can be quickly located by testing a sample of milk representing a mixture of the four quarters of the cow which is a composite sample. Following location of problem animals at one milking, these can be checked again at the next milking by carrying out a test on each quarter separately. This can be achieved by using a sampling device designed to take separate samples of each teat of the cow's udder simultaneously and placing each sample in a set of four square reaction cuvettes.

A first substrate may comprise 68 mg. of pNP-NAG to which has been added 2 ml. of ethanol. After shaking 8 ml. of 1M citrate buffer at pH 4.3 may be added to provide a 20mM pNP-NAG solution. In this solution calculated on a basis of per 100g. $H_2O$ and an equal volume of milk to be added there is present 6% substrate, 65% citrate and 29% citric acid. However, in this specific case it should be remembered that 20% of ethanol is present in the 100 g. $H_2O$ criterion.

Alternatively a second substrate may comprise 68 mg pNP-NAG in 4.0 ml. of 1M pH 4.5 citrate buffer. This may comprise 1.5% substrate, 68% citrate and 30.5% citric acid per 100 g. $H_2O$ and on an equal volume of milk to be added.

In the case of the first substrate solution a small volume (eg. 0.05 ml) can be added directly to 0.45-0.50 ml of milk sample contained in suitable reaction vessels such as test tubes or cuvettes. The following discussion concerns a detailed assay method using the first substrate.

<u>EQUIPMENT NEEDED</u>

(i) It may be necessary to firstly collect samples in separate bottles then transfer 0.45-0.50 ml. of each to the reaction cuvettes. Alternatively, a sampling device may be used as referred to above.

(ii) Reaction tubes - preferably sets of 4 square (1 cm x 1 cm) cuvettes capable of holding 3-4 mls.

(iii) Disposable pipettes.

(iv) It may be advantageous to have a small portable incubator capable of holding samples at 37°C for up to 10 mins. A polyurethane block specially cut to hold the set of 4 cuvettes would be adequate.

(v) The substrate vials could be fitted with a screw cap containing an "eye-dropper" which delivered approximately 0.05 ml. of solution with each drop (see Method below).

<u>METHOD</u>

(a) Collect or transfer 0.45-0.50 ml. of milk into each of the four cuvettes. Ensure that the temperature of the milk is around 37°C. If all composites have been collected first before testing started, then rewarm them back to 37°C if they had cooled down.

(b) Add 0.05 ml. (or 1 drop) of the warmed (37°C) substrate solution and mix.

(c) Place in suitable incubator (see previous comments) and stand for -

(i) 5 mins. if examining 4 quarter samples

NOTE; Always test quarters from the same animal in the 4 cuvette system)

(ii) 10 mins. if examining composite samples.

(d) Stop each reaction with 2.5 mls. of 2% $Na_4EDTA$ and mix each set of tubes by inverting a few times.

For quarter samples, the best way of deciding if one or more quarters are mastitic is to look for an imbalance in the colours of the 4 cuvettes. Negative quarters are usually colourless or slightly coloured (yellow), while mastitic quarters are definitely yellow. If all quarters are definitely yellow (and to the same intensity) it means that all quarters are infected or that there is some other physiological disturbance which is causing an abnormal secretion in all quarters. Cows showing this pattern should be watched closely to see if the secretory disturbance "rights" itself or if indeed it is really a widespread mastitic infection.

For composite samples, a quick visual observation of a set of tests (say 40 - i.e. 10 sets of cuvettes) will enable an operator to pick out the worst animals. Closer observation with a set of colour standards to refer to, will enable the more marginal animals to be located. These animals can then be checked next milking by examining their individual quarter samples.

Although the procedure outlined above works well some improvements could be made by changing the substrate to 4-methylumbelliferyl NAG which when hydrolysed by NAGase becomes fluorescent. Because of the greater sensitivity of this fluorescent substrate, the reaction time could be reduced dramatically (to say 1-2 mins. for quarter sample analyses and 4-5 mins. for composite sample analyses). The only other apparatus needed would be a portable light source which emitts light of a wave length in the range 350-380 nm.

In the case of the second substrate 0.2 ml. aliquots of substrate-buffer was transferred into a plurality of cuvettes and freeze-dried. When required to be used 0.4-0.5 ml. of milk was transferred to each cuvette and each cuvette then shaken.

0097506

In the case of both the first and second substrates a suitable stopping agent may be 2 vials each containing 5 g. of $Na_4EDTA$ which may be dissolved in 250 ml. of $H_2O$. Standard vials may also be used as described previously. However, if desired it may be possible to provide a card showing a series of increasing intensities of the yellow colour with which the final test colours may be compared.

It will also be appreciated that instead of containers containing one or more reaction system components such as cuvettes or vials a suitable supporting medium may be used. Materials which may form the supporting medium are those materials which by means of capillary action or any other physical chemical technique are able to hold liquids. Examples include filter paper, fibrous material including cellulosic material, wood strips, felt, porous ceramic strips and cotton substrates.

0097506

CLAIMS

1. A reaction system for diagnosis of mastitis using the enzyme NAGase including the components

(A) a substrate for reaction with the enzyme NAGase selected from a compound of the formula

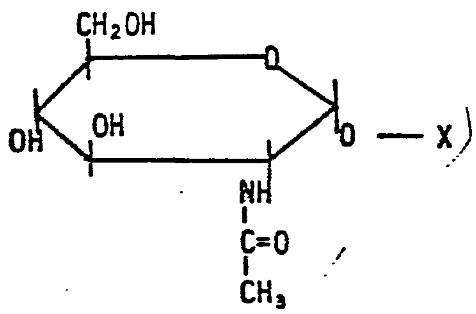

where X is selected from p-nitrophenol, α-naphthol, p-nitrocatechol, 3-3 bis (p-hydroxyphenyl) phthalide, 2-methoxy-4-(2'-nitrovinyl)-phenyl, and 4 methyl umbelliferone, and

(B) a buffer for component A to maintain the pH of the substrate within the range of 3.5 to 6.0.

2. A reaction system as claimed in claim 1 wherein the buffer is in the form of an acid/anion mixture selected from citric acid/citrate, acetic acid/acetate, formic acid/formate and lactic acid/lactate.

3. A reaction system as claimed in claim 2 wherein the component A is present in the reaction mixture in an amount of 0.5 to 2.5%, the buffer anion is present in an amount of from 50 to 75.0% and the buffer acid is present in an amount of 20 to 40% wherein the above amounts are calculated on the basis that the reaction mixture is present in 100g of $H_2O$ and that for each volume of reaction mixture there is also used an equal volume of milk.

4. A reaction system as used in claim 3 wherein the amount of component A is 1.0 to 1.5%, the buffer anion is present in an amount of 65-70% and the buffer acid is present in an amount of 25-30%.

5. A reaction system as claimed in claim 4 wherein the amount of component A is 1.3%, the amount of buffer anion is 68.0% and the amount of buffer acid is 30.0%.

6. A reaction system as claimed in any preceding claim also including a stopping agent which stops the reaction between

substrate and enzyme by raising the pH to a value of between 8 and 11.

7. A reaction system as claimed in claim 6 wherein the stopping agent is selected from the sodium salt of EDTA, glycine, NaOH, sodium borate and the sodium carbonate.

8. A reaction system as claimed in any preceding claim wherein the reaction system includes standards selected from p-nitrophenol, α-naphthol, p-nitrocatechol, 3-3 bis (p-hydroxyphenyl) phthalide, 2-methoxy-4-(2'-nitrovinyl)- phenyl and 4 methyl umbelliferone.

9. A reaction system as claimed in any preceding claim in the form of a kit containing predetermined amounts of substrate-buffer, standards and stopping agent in separate vials, containers or support media.

10. A method of diagnosis of mastitis using the reaction kit of claim 9 including the steps of:

(1) addition of water to substrate buffer followed by heating;

(2) addition of milk sample to a reaction vessel containing substrate buffer;

(3) incubating for a suitable period, and

(4) addition of a stopping agent followed by reading colour in a colour comparator vessel or against a prepared set of standards.